(19) Europäisches Patentamt
European Patent Office
Office européen des brevets

(11) Veröffentlichungsnummer: **0 036 161**
**B1**

(12) # EUROPÄISCHE PATENTSCHRIFT

(45) Veröffentlichungstag der Patentschrift: **10.08.83**

(51) Int. Cl.³: **C 07 C 99/10**

(21) Anmeldenummer: **81101715.1**

(22) Anmeldetag: **09.03.81**

(54) **Verfahren zur Herstellung von Alkalisalzen des Phenylglycins.**

(30) Priorität: **19.03.80 DE 3010511**

(43) Veröffentlichungstag der Anmeldung: **23.09.81 Patentblatt 81/38**

(45) Bekanntmachung des Hinweises auf die Patenterteilung: **10.08.83 Patentblatt 83/32**

(84) Benannte Vertragsstaaten: **BE CH DE FR GB LI**

(56) Entgegenhaltungen:
**DE-A-1 543 342**
**DE-A-2 821 728**
**DE-C-135 332**
**DE-C-145 376**
**GB-A-1 119 256**

(73) Patentinhaber: **BASF Aktiengesellschaft, Carl-Bosch-Strasse 38, D-6700 Ludwigshafen (DE)**

(72) Erfinder: **Distler, Harry, Dr., In den Hahndornen 5, D-6719 Bobenheim (DE)**
Erfinder: **Steingruber, Elmar, Dr., Amselweg 44, D-6710 Frankenthal (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# Verfahren zur Herstellung von Alkalisalzen des Phenylglycins

Die Erfindung betrtifft ein Verfahren zur Herstellung von Alkalisalzen des Phenylglycins durch Umsetzung von Anilin mit Formaldehyd und Blausäure unter bestimmten Reaktionsbedingungen bezüglich Temperatur, Reaktionszeit und Blausäurekonzentration und Hydrolyse des Phenylglycins in Abwesenheit oder in Gegenwart von Anilin in einer Menge zwischen 0 und 0,2 Mol Anilin je Mol Phenylglycinnitril mit Alkalilaugen in einem Verhältnis von 1 bis 1,05 Mol Alkalihydroxid je Mol Phenylglycinnitril in Gegenwart von Alkoholen, aromatischen Kohlenwasserstoffen und/oder aliphatischen Kohlenwasserstoffen als organischem Lösungsmittel in einer Menge von 5 bis 200 Gewichtsprozent und von Wasser in einer Menge von 50 bis 500 Gewichtsprozent, bezogen auf Ausgangsanilin.

Es ist aus der deutschen Patentschrift 1 543 342 bekannt, Anilin mit Formaldehyd und Blausäure bei einer Temperatur zwischen 80 und 130°C kontinuierlich umzusetzen und das Reaktionsgemisch mit Alkalihydroxid zu verseifen. Das Phenylglycinnitril selbst wird nicht isoliert. Es wird angegeben, dass die Blausäure und der Formaldehyd im allgemeinen im Reaktionsgemisch zu weniger als 10%, häufig nur zu 1 bis 5% in freier Form vorliegt. In allen Beispielen wird keine freie Blausäure angewendet. Die Hydrolyse des gebildeten Phenylglycins wird kontinuierlich nach der Umsetzung mit Natronlauge, Kalilauge oder deren Gemischen und mit 1 bis 1,3 Mol Alkalihydroxid je Mol Phenylglycinnitril durchgeführt. Das Verseifungsgemisch enthält 40 bis 80 Gewichtsprozent Wasser. Bei Umsetzung und Hydrolyse werden keine zusätzlichen organischen Lösungsmittel verwendet. In den Beispielen wird mit einem 50-gewichtsprozentigen Laugengemisch (60 KOH : 40 NaOH) bei Siedetemperatur verseift. Es wird darauf hingewiesen, dass das Reaktionsgemisch der Nitrilherstellung einen Anilinüberschuss enthält, der durch Extraktion mit Benzol oder Toluol oder durch Abdestillation des Anilins als Azeotrop mit Wasser entfernt wird. Diese azeotrope Destillation des Anilins mit Wasser kann auch während der Verseifung oder Nachreaktion durchgeführt werden. Die Abtrennung des Anilins erfolgt nach der Hydrolyse in Beispiel 1 durch Extraktion, in Beispiel 2 durch azeotrope Destillation von Anilin und Wasser. Wie jedoch schon die deutsche Offenlegungsschrift 2 625 935 feststellt, ist bei der Herstellung, insbesondere im grosstechnischen Massstab, dem Endstoff stets ein wesentlicher Anteil an nicht umgesetztem Anilin beigemengt. Die Beimengung tritt bei der Verseifung zum Kaliumsalz des Phenylglycins erneut als Verunreinigung auf. Zur Isolierung von Phenylglycinalkalisalzen müssen Anilin und harzige Nebenprodukte entfernt werden, was nur teilweise durch eine Extraktion mit einem inerten Lösungsmittel wie Cyclohexan, Benzol oder Toluol gelingt. Das Verfahren ist mit Bezug auf einfache und wirtschaftliche Aufarbeitung und gute Ausbeute unbefriedigend.

Es ist aus BIOS Final Report Nr. 986, Seiten 312 bis 316, bekannt, die Umsetzung mit 2 Mol Anilin je Mol Formaldehyd und die Hydrolyse in Gegenwart überschüssiger Mengen an Anilin mit einem wässrigen Laugengemisch bei 110°C durchzuführen. Man bestimmt den Phenylglycingehalt in der Hydrolyselösung durch Titration mit Natriumnitritlösung. Es ist nachteilig, dass man von einem mindestens 1-molaren Anilinüberschuss ausgehen muss, grosse Mengen verdünnter Natriumhydrogencarbonatlösung als Nebenprodukt anfallen, man das Verfahrensprodukt in relativ verdünnter Lösung erhält und so sehr grosse Lager- und Reaktionsgefässe erforderlich sind.

Es ist aus der deutschen Offenlegungsschrift 2 621 449 und der deutschen Patentschrift 2 621 728 bekannt, dass man Phenylglycinnitril durch Umsetzung von Formaldehyd mit Anilin und Blausäure in Gegenwart von Wasser während 0,1 bis 4 Stunden bei einer Temperatur von 0 bis 80°C herstellen kann, wobei die Konzentration der Blausäure während der Reaktion nicht mehr als 0,9 Gewichtsprozent, bezogen auf das Reaktionsgemisch, beträgt. Bevorzugt verwendet man Wasser allein als Lösungsmittel, gegebenenfalls auch unter den Reaktionsbedingungen inerte, organische Lösungsmittel. In den Beispielen dieser Veröffentlichungen wird ohne Verwendung organischer Lösungsmittel umgesetzt. Es wird ausdrücklich darauf hingewiesen, dass der Endstoff aus dem Reaktionsgemisch in üblicher Weise, z.B. durch Destillation oder Extraktion mit z.B. Cyclohexan und Destillation des Lösungsmittels, isoliert wird.

Es ist aus Ullmanns Encyklopädie der technischen Chemie, (3. Auflage), Band 8, Seiten 214 und 215 bekannt, dass man das bei der Herstellung von Phenylglycinnitril anfallende, rohe Gemisch von Nitril und unumgesetztem Ausgangsanilin von der wässrigen Phase des Herstellungsgemisches abtrennt und mit einem äquimolekularen Gemisch von Kali- und Natronlauge bei etwa 110°C verseift. Das ausgeschiedene Anilin wird abgetrennt und kehrt in den Prozess zurück. Die Phenylglycin-Lösung, die noch beträchtliche Mengen Anilin enthält, wird kontinuierlich mit Benzol extrahiert und im Vakuum auf 55% eingeengt. Das Konzentrat dampft man auf Vakuum-Walzentrocknern zur Trockene ein. Auch diese Veröffentlichung nennt als analytische Bestimmung des Phenylglycingehaltes die Titration mit Natriumnitrit. In der deutschen Auslegeschrift 2 717 371 wird ein Verfahren zur Herstellung von rieselfähigen Alkalisalzen des Phenylglycins durch stufenweise Verseifung des Nitrils mit wässrigen Alkalilaugen in Gegenwart von Anilin bei Temperaturen zwischen 50°C und der Siedetemperatur und Eindampfen des Reaktionsgemisches beschrieben, wobei man für die Verseifung Mutterlaugen verwendet, die bei Alkalihydroxid/Alkaliamid-Schmelzen anfallen und die –

bezogen auf die Mutterlauge – 7 bis 30 Gew.% Natrium-/Kaliumhydroxid-Gemisch, bis zu 20 Gew.% Natrium-/Kaliumcarbonat sowie gegebenenfalls bis zu 10 Gew.% weitere Bestandteile neben kleineren Mengen an farbigen Bestandteilen und organischen Zersetzungsprodukten enthalten. Das Verfahren wird im allgemeinen so durchgeführt, dass das Phenylglycinnitril in Form der Lösung in Anilin in die Abfallauge eingetragen wird und die Verseifung zunächst bei 40 bis 70°C begonnen wird. Nach einer Stunde bei dieser Temperatur wird dann das Reaktionsgemisch zum Sieden erhitzt bis alles Nitril verseift ist. Die Verseifung erfolgt entsprechend den Angaben in der GB-PS 1 119 256. Nach dem Abkühlen wird das Anilin mit Toluol extrahiert und die wässrige Phenylglycinsalzlösung in üblicher Weise zur Trockene eingedampft.

Es ist aus der britischen Patentschrift 1 119 256 bekannt, dass man zur Herstellung von Phenylglycinsalz durch Umsetzung von Anilin mit Hydroxyacetonitril zu Phenylglycinnitril und anschliessende Hydrolyse die wässrig-alkalische Hydrolyse des Phenylglycinnitrils in Gegenwart von 0,05 bis 0,6 Mol, vorzugsweise 0,2 bis 0,5 Mol, Anilin pro Mol Phenylglycinnitril vornimmt. Auch bei diesem Verfahren wird das Phenylglycinnitril ohne zusätzliches Lösungsmittel hydrolysiert. Es wird ausdrücklich darauf hingewiesen, dass bei einer Hydrolyse von rohem Phenylglycinnitril ohne Beimengung von Anilin bzw. in Anwesenheit sehr geringer Mengen von Anilin, keineswegs der Reinheitsgrad des Phenylglycinsalzes erhöht wird. Den Reinheitsgrad bestimmen nach der Lehre der britischen Patentschrift anwesende störende Nebenprodukte, die bei der Hydrolyse anilinfreien Nitrils ungenügend bis mässig reines Phenylglycinsalz und bei der Hydrolyse von Anilin und Phenylglycinnitril in einem Molverhältnis von 0,05:1 eine mässige bis genügende Reinheit der Salze ergeben. So erklärt sich auch, dass bei diesem Verfahren ein Molverhältnis von mindestens 0,2:1 (Anilin:Phenylglycinnitril) bei der Hyrolyse bevorzugt ist, da erst so eine gute (ab einem Molverhältnis 0,5:1 sehr gute) Reinheit des Salzes erzielt wird. Insgesamt wird eine Anwesenheit von Anilin in vorgenannten Mengen verlangt, um neben der Reinheit auch eine gute Ausbeute zu erzielen.

Die deutsche Offenlegungsschrift 2 625 935 zeigt, dass bei der Verseifung in Anwesenheit von Anilin stets Verunreinigungen auftreten. Daneben wird ebenfalls auf harzige Nebenprodukte hingewiesen, die sich bei der Herstellung des Phenylglycins infolge der Anwesenheit von Formaldehyd bilden. Diese Verunreinigungen werden durch die üblichen Verfahren nicht völlig entfernt und man sieht eine Lösung der Schwierigkeiten darin, das rohe Phenylglycinnitril nach der Herstellung mit einer Säure bei einem pH zwischen 1 und 7 zu behandeln.

Es wurde nun gefunden, dass man Alkalisalze des Phenylglycins durch Umsetzung von Anilin mit Formaldehyd und Blausäure in Gegenwart von Wasser während 0,1 bis 4 Stunden bei einer Temperatur von 0 bis 80°C, wobei die Konzentration der Blausäure während der Reaktion nicht mehr als 0,9 Gewichtsprozent, bezogen auf das Reaktionsgemisch, beträgt und Hydrolyse des gebildeten Phenylglycinnitrils vorteilhaft erhält, wenn die Hydrolyse in Abwesenheit oder in Gegenwart von Anilin in einer Menge zwischen 0 und 0,2 Mol Anilin je Mol Phenylglycinnitril mit Alkalilaugen in einem Verhältnis von 1 bis 1,05 Mol Alkalihydroxid je Mol Phenylglycinnitrit in Gegenwart von Alkoholen, aromatischen Kohlenwasserstoffen und/oder aliphatischen Kohlenwasserstoffen als organischem Lösungsmittel in einer Menge von 5 bis 200 Gewichtsprozent und von Wasser in einer Menge von 50 bis 500 Gewichtsprozent, bezogen auf Ausgangsanilin, durchgeführt wird.

Die Umsetzung und Hydrolyse können für den Fall der Verwendung von Kalilauge durch die folgenden Formeln wiedergegeben werden:

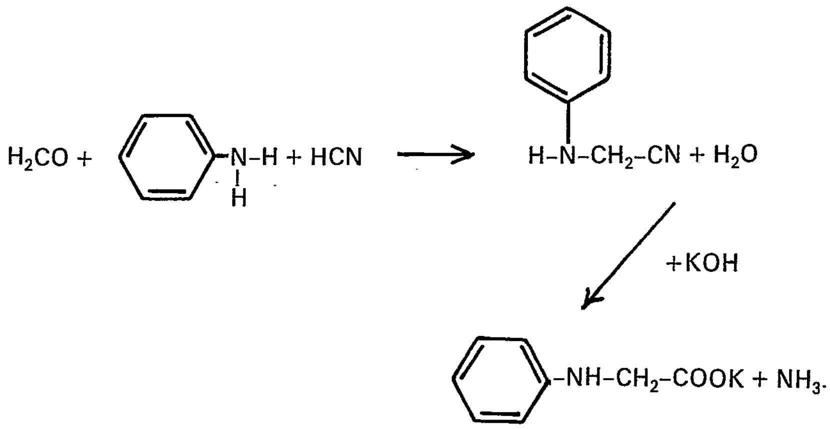


Im Vergleich zu den bekannten Verfahren, insbesondere zu dem in der deutschen Offenlegungsschrift 1 543 342 beschriebenen Verfahren, liefert das Verfahren nach der Erfindung auf einfacherem und wirtschaftlicherem Wege Alkalisalze des Phenylglycins in besserer Ausbeute und Reinheit. Das Verfahren ist gerade für den Betrieb im grosstechnischen Massstab und für einen kontinuierlichen Betrieb geeignet, bietet keine wesentlichen Abwasserfragen und ist praktisch frei von

harzigen Nebenprodukten, welche sich durch Anwesenheit höherer Mengen an Anilin bei der Umsetzung und Hydrolyse oder bei höherer Reaktionstemperatur infolge der Anwesenheit von Formaldehyd bevorzugt bilden. Aufwendige Reinigungsoperationen durch nachträgliche Extraktion des Anilins werden eingespart. Ebenfalls liefern die Salze bei Folgesynthesen, insbesondere der Indigosynthese, entsprechend reinere Folgeprodukte.

Alle diese vorteilhaften Eigenschaften sind im Hinblick auf den Stand der Technik überraschend. Wie Ullmann (loc. cit., Band 3, Seiten 494 bis 496) zeigt, bildet sich im alkalischen Medium aus Anilin und Formaldehyd besonders leicht Methylendiphenyldiimid. Solche und ähnlich gebaute polymere Anilinharze sind unerwünschte Nebenprodukte, welche den Ablauf des technischen Indigoprozesses empfindlich stören können. Diese Nachteile fallen besonders im grosstechnischen, kontinuierlichen Betrieb ins Gewicht, denn die Umsetzungsprodukte von Anilin, Formaldehyd und Blausäure werden sofort und ohne weitere Reinigung der Hydrolyse zugeführt. Weiterhin ist die Instabilität des Anilins, vor allem oxidativen Einflüssen gegenüber, bekannt [Kirk-Othmer - Interscience Encyclopedia (New York 1947), Band 1, Seiten 914 und 915). Harzartige und andere schwer aufzuklärende Nebenprodukte sind technisch schwer abtrennbar, stören den Indigoprozess erheblich und wirken sich empfindlich auf die Qualität des Indigo aus.

Man konnte im Hinblick auf die deutsche Offenlegungsschrift 2625935 auch nicht erwarten, dass die erfindungsgemässe Hydrolyse ohne Säurebehandlung und sogar in Anwesenheit geringer Anilinmengen die vorteilhaften Ergebnisse liefert. Ebenfalls war im Hinblick auf die deutsche Offenlegungsschrift 1543342 zu vermuten, dass ohne Abtrennung von Anilin vor der Hydrolyse mittels Extraktion bzw. Destillation nur ein unreineres Glycinsalz in schlechterer Ausbeute erhalten würde. Andererseits musste auch im Hinblick auf die britische Patentschrift angesichts der Abwesenheit bzw. der Anwesenheit von Anilin in geringeren Mengen bei der Hydrolyse ein unreinerer Endstoff in noch dazu wesentlich schlechterer Ausbeute erwartet werden. Mit Bezug auf den gesamten Stand der Technik war es darüber hinaus überraschend, dass gerade die erfindungsgemässen organischen Lösungsmittel und die erfindungsgemässen Mengenverhältnisse an Lösungsmittel, Wasser und Alkali, bezogen auf Ausgangsanilin, die vorteilhaften Ergebnisse liefern.

Die bessere Reinheit der Endstoffe kann insbesondere durch eine genauere Analyse des Hydrolysengemischs bzw. des Endstoffs unter Verwendung der Hochdruck-Flüssigkeits-Chromatographie anstelle der früheren Methoden, insbesondere der Natriumnitrit-Titration, gezeigt werden. Bezüglich der Durchführung dieser Analyse wird auf Heinz Engelhardt, Hochdruck-Flüssigkeits-Chromatographie (Springer-Verlag, Heidelberg, 1977) verwiesen.

Formaldehyd kann in flüssiger Form oder als Gas, im allgemeinen in Form seiner wässrigen, zweckmässig von 10- bis 50-, vorzugsweise von 30- bis 40-gewichtsprozentigen Lösung verwendet werden. Blausäure kommt als Gas oder zweckmässig in flüssiger Form oder in wässriger Lösung in Betracht. Anilin kann bevorzugt allein oder in Lösung, zweckmässig in einem organischen Lösungsmittel, verwendet werden. Zweckmässig sind 40- bis 60-gewichtsprozentige Lösungen. Man kann die drei Ausgangsstoffe in stöchiometrischer Menge oder jede der Komponenten jeweils im Überschuss umsetzen, vorzugsweise in einem Überschuss über die stöchiometrische Menge von 0,01 bis 0,15 Mol Anilin, vorzugsweise 0,01 bis 0,05 Mol Anilin und/oder von 0,01 bis 0,1 Mol Blausäure je Mol Formaldehyd (berechnet 100%).

Die Umsetzung wird im allgemeinen bei einer Temperatur von 0 bis 80, zweckmässig zwischen 40 und 80°C, vorzugsweise von 45 bis 75°C, insbesondere von 50 bis 70°C, mit Unterdruck oder Überdruck oder vorzugsweise drucklos, diskontinuierlich oder vorzugsweise kontinuierlich durchgeführt. Man verwendet Wasser, zweckmässig in Gestalt von wässriger Formaldehydlösung und/oder Anilinlösungen, daneben bildet sich bei der Reaktion selbst Wasser; insgesamt kommt eine Gesamtmenge von 1 bis 6, vorzugsweise von 3 bis 4 Mol Wasser, bezogen auf Carbonylverbindung, in Betracht. Blausäure wird dem Ausgangsgemisch vor und während der Reaktion in einer solchen Menge zugegeben, dass die Konzentration während der Reaktion nicht mehr als 0,9, im allgemeinen von 0,01 bis 0,9, vorteilhaft von 0,01 bis 0,8, zweckmässig von 0,01 bis 0,7, bevorzugt von 0,01 bis 0,1, insbesondere von 0,05 bis 0,1 Gewichtsprozent Blausäure, bezogen auf das Reaktionsgemisch, beträgt. Die Reaktionszeit (im kontinuierlichen Betrieb Verweilzeit) beträgt 0,1 bis 4, vorzugsweise 1 bis 2 Stunden. Man verwendet Wasser allein als Lösungsmittel oder bevorzugt zusammen mit unter den Reaktionsbedingungen inerten, organischen Lösungsmitteln, die zweckmässig den bei der Hydrolyse verwendeten Lösungsmitteln entsprechen. Es kommen in Frage: aromatische Kohlenwasserstoffe, z.B. Toluol, Benzol, Äthylbenzol, o-, m-, p-Xylol, Isopropylbenzol, Methylnaphthalin; aliphatische oder cycloaliphatische Kohlenwasserstoffe, z.B. Heptan, α-Pinen, Pinan, Nonan, Benzinfraktionen innerhalb eines Siedepunktintervalls von 70 bis 190°C, Cyclohexan, Methylcyclohexan, Petroläther, Dekalin, Hexan, Ligroin, 2,2,4-Trimethylpentan, 2,2,3-Trimethylpentan, 2,3,3-Trimethylpentan, Octan; Alkohole, n-Butanol, Isobutanol, tert.-Butanol, Glykol, Glycerin, n-Propanol, Isopropanol, Amylalkohol, Cyclohexanol, 2-Methyl-4-pentanol, Äthylenglykolmonoäthyläther, 2-Äthylhexanol, Methylglykol, n-Hexanol, Isohexylalkohol, Isoheptylalkohol, n-Heptanol, Äthylbutanol, Nonylalkohol, Dodecylalkohol, Methylcyclohexanol, insbesondere solche mit 1 bis 12 Kohlenstoffatomen; und entsprechende Gemische; gegebenenfalls in einer Menge von 40 bis 10000

Gewichtsprozent, z.B. von 50 bis 1500 Gewichtsprozent, bezogen auf Formaldehyd, zweckmässiger jedoch in einer Menge, die dem vorgenannten erfindungsgemässen Mengenverhältnis an organischem Lösungsmittel, bezogen auf Ausgangsanilin, entspricht.

Die Reaktion kann wie folgt durchgeführt werden: Ein Gemisch von Formaldehyd, Blausäure und Anilin, zusammen mit Wasser und dem organischen Lösungsmittel, wird während der Reaktionszeit bei der Reaktionstemperatur gehalten. Man gibt Anteile der Blausäure in das Ausgangsgemisch und während der Reaktion in Portionen bzw. kontinuierlich so zu, dass die vorgenannte Blausäurekonzentration während der gesamten Reaktioszeit eingehalten wird. Die laufende Messung der Blausäurekonzentration erfolgt zweckmässig über eine Silber-Kalomelelektrode. Dann wird das Reaktionsgemisch der Hydrolyse in Gestalt eines weiteren Hydrolysegefässes zugeführt oder zweckmässig dem Reaktionsgemisch die entsprechenden Mengen an der für die Hydrolyse notwendigen Komponenten zugeführt. So kann man z.B. aus dem Reaktionsgemisch Anteile an Wasser und/oder organischem Lösungsmittel abziehen oder dem Gemisch zusetzen. Die Reaktion wird zweckmässig jedoch so durchgeführt, dass die für die Hydrolyse notwendigen Mengen an Wasser, organischem Lösungsmittel und Anilin schon im Ausgangsgemisch bzw. Reaktionsgemisch vorhanden sind.

Die Menge an Anilin beträgt bei Beginn der Hydrolyse von 0 bis 0,2, zweckmässig 0 bis 0,1, vorteilhaft 0 bis 0,05, bevorzugt 0 bis 0,03, insbesondere 0 bis 0,01 Mol Anilin je Mol Phenylglycinnitril. Bei einer besonders bevorzugten Ausgangsform wird das Reaktionsgemisch des Phenylglycinnitrils in Abwesenheit von unumgesetztem Anilin hydrolysiert. Das erfindungsgemässe Wasser bei der Hydrolyse wird als das Gesamtwasser definiert, das sowohl das Wasser des Ausgangsgemischs, das bei der Reaktion gebildete Wasser, gegebenenfalls während der Reaktion und Hydrolyse zugesetztes Wasser und das mit der Lauge zugeführte Wasser umfasst. Gegebenenfalls kann man während der Hydrolyse einen Anteil an Wasser, gegebenenfalls zusammen mit einem Anteil an organischem Lösungsmittel, abdestillieren. Jedoch werden von Anfang bis Ende der Hydrolyse im Hydrolysengefäss die anwesenden Mengen an Wasser und organischem Lösungsmittel trotz Zugabe der Abdestillation von Anteilen dieser Komponenten innerhalb der vorgenannten, erfindungsgemässen Mengenverhältnisse eingestellt. Bei der Hydrolyse verwendet man eine Menge von 50 bis 500, insbesondere 100 bis 200 Gewichtsprozent Wasser und eine Menge von 5 bis 200, insbesondere 10 bis 100 Gewichtsprozent organischem Lösungsmittel, bezogen auf Ausgangsanilin. Ausgangsanilin wird als die dem Ausgangsgemisch der Phenylglycinnitrilherstellung zugeführte Anilinmenge definiert. Man verwendet als organisches Lösungsmittel bei der Hydrolyse Alkohole und vorteilhaft aliphatische Kohlenwasserstoffe und bevorzugt aromatische Kohlenwasserstoffe, z.B. die schon im Falle der Phenylglycinnitrilherstellung genannten, bevorzugt Toluol, Benzol, Xylole.

Die Hydrolyse wird in der Regel bei einer Temperatur von 50 bis 150°C, insbesondere 55 bis 110°C, drucklos oder unter Druck, diskontinuierlich oder vorzugsweise kontinuierlich, zweckmässig während 1 bis 10, insbesondere 2 bis 3 Stunden durchgeführt. Als Alkalilaugen verwendet man vorteilhaft Natronlauge oder Kalilauge, zweckmässig ein Gemisch dieser Laugen, vorzugsweise 0,2 bis 0,8, insbesondere 0,4 bis 0,5 Mol KOH je Mol NaOH. Zweckmässig sind 5- bis 50-, insbesondere 30- bis 40-gewichtsprozentige, wässrige Laugen, vorteilhaft NaOH/KOH-Laugengemische. Man verwendet 1 bis 1,05, insbesondere 1 bis 1,01 Mol Alkalihydroxid, besonders ein Gemisch von Natriumhydroxid und Kaliumhydroxid, je Mol Phenylglycinnitril.

Die Hydrolyse kann wie folgt durchgeführt werden: Dem Reaktionsgemisch der Nitrilherstellung wird Lauge, zweckmässig das vorgenannte Laugengemisch zugegeben, gegebenenfalls werden die Gesamtmengen an Wasser und organischem Lösungsmittel noch auf die erfindungsgemässen Mengen eingestellt und dann das Hydrolysengemisch während der Reaktionszeit bei der Reaktionstemperatur gehalten. Nach Ende der Hydrolyse wird bevorzugt das organische Lösungsmittel und Wasser, gegebenenfalls mit der Restmenge an Anilin, abdestilliert und der Endstoff in üblicher Weise, z.B. durch Einengen des Gemischs und Filtration, isoliert.

Die nach dem Verfahren der Erfindung erhältlichen N-Phenylglycinsalze sind wertvolle Ausgangsstoffe für die Herstellung von Farbstoffen, Fungiciden, Bactericiden, Textilhilfsmitteln, Alterungsschutzmitteln und Inhibitoren von Frostschutzmitteln. Die Alkalisalze des Phenylglycins sind Ausgangsstoffe für die Indigosynthese. Bezüglich der Verwendung wird auf die vorgenannten Veröffentlichungen und Ullmanns Encyklopädie der technischen Chemie, Band 9, Seite 388, Band 15, Seite 219, Band 19, Seite 300, 317, 339, verwiesen.

Die in den folgenden Beispielen aufgeführten Teile bedeuten Gewichtsteile.

Beispiel 1

In einem Rührkessel werden bei 65°C stündlich zu 350 Teilen Toluol 400 Teile 30-gewichtsprozentige, wässrige Formaldehydlösung, 108 Teile flüssige Blausäure und 372 Teile Anilin langsam zugegeben, wobei im Reaktionsraum die Blausäurekonzentration von 0,1 Gewichtsprozent (bezogen auf das Reaktionsgemisch) nicht überschritten wird. Die durchschnittliche Blausäurekonzentration beträgt 0,08 Gewichtsprozent. Nach einer mittleren Verweilzeit von 60 Minuten wird das Reaktionsgemisch in einen Reaktor geführt, dessen Temperatur bei 70°C liegt. Im Reaktor beträgt die mittlere Verweilzeit 60 Minuten. Die Blausäurekonzentration liegt durchschnittlich bei 0,04 Gewichtsprozent.

Nach Abkühlen des Gemischs auf 45°C gibt man ein Gemisch von 152 Teilen 50-gewichtsprozentiger, wässriger Natronlauge, 213 Teile 50-gewichtsprozentiger, wässriger Kalilauge und 120 Teile Wasser während 0,5 Stunden zu, wobei die Temperatur auf 55°C ansteigt. Man erhitzt während einer Stunde auf 60°C. Nun setzt die Hydrolyse in exothermer Reaktion ein. Man lässt die Temperatur während einer Stunde am Rückfluss bis 80°C ansteigen und hält das Gemisch während einer Stunde am Rückfluss. Die Hydrolyse ist jetzt beendet. Man destilliert nun über einen Auskreiser die Gesamtmenge an Toluol und 3 Teile Anilin als azeotropes $H_2O$/Toluol/Anilin-Gemisch ab. Die klare, nahezu farblose, wässrige Salzlösung wird im Vakuum eingeengt, wobei 650 Teile Wasser abdestillieren und der Endstoff ausfällt. Man erhält stündlich 712 Teile Natrium/Kaliumsalz des Phenylglycins vom Fp 140 bis 160°C (Zers.) und einer Reinheit von 96 Prozent (gemessen durch Hochdruck-Flüssigkeits-Chromatographie), entsprechend 695 Teile reinem Endstoff (96% der Theorie).

Beispiel 2 (Vergleich)

In einem Rührgefäss werden analog Beispiel 2 der GB-PS 1 119 256 1395 Teile Anilin mit 1212 Teilen einer 47-gewichtsprozentigen, wässrigen Lösung von stabilisiertem Hydroxyacetonitril und 600 Teilen Wasser innerhalb von 30 Minuten auf 100°C gebracht. Das Reaktionsgemisch wird 2 Stunden unter Rühren bei 100 bis 110°C gehalten und danach auf 40 bis 50°C abgekühlt. Bei dieser Temperatur werden 1030 Teile einer 50-gewichtsprozentigen Kali/Natronlauge (Gewichtsverhältnis KOH:NaOH 58:42) zugesetzt. Die Hydrolyse wird binnen 60 Minuten bei 50 bis 65°C und dann 2 Stunden bei Siedetemperatur durchgeführt. Nach beendeter Reaktion und Abkühlen auf Raumtemperatur wird das Anilin mit Benzol extrahiert und die Phenylglycinsalzlösung eingedampft. Man erhält 1755 Teile Phenylglycinsalz als rohes etwas braunstichiges Salz, das nach dem Befund unter Verwendung der Hochdruck-Flüssigkeits-Chromatographie noch deutliche Verunreinigungen aufweist. Nach Umkristallisieren aus Wasser erhält man 1697 Teile Salz mit einer noch leichten braunstichigen Tönung und einer Reinheit (Befund unter Verwendung der Hochdruck-Flüssigkeits-Chromatographie) von 89 Prozent, entsprechend 83% der Theorie.

Beispiel 3 (Vergleich)

In einem Rührkessel werden bei 65°C stündlich 400 Teile 30-gewichtsprozentige, wässrige Formaldehydlösung, 108 Teile flüssige Blausäure und 372 Teile Anilin langsam zugegeben, wobei im Reaktionsraum die Blausäurekonzentration von 0,1 Gewichtsprozent (bezogen auf das Reaktionsgemisch), nicht überschritten wird. Die durchschnittliche Blausäurekonzentration beträgt 0,08 Gewichtsprozent. Nach einer mittleren Verweilzeit von 60 Minuten wird das Reaktionsgemisch in einen Reaktor geführt, dessen Temperatur bei 65°C liegt. Im Reaktor beträgt die mittlere Verweilzeit 45 Minuten. Die Blausäurekonzentration liegt durchschnittlich bei 0,04 Gewichtsprozent. Man erhält stündlich 880 Teile Reaktionsgemisch, die in einen Verseifungskessel, wo stündlich 200 Teile Wasser und 548 Teile 30-gewichtsprozentige Natronlauge bei 45°C eingebracht werden, fliessen. Nach einer Verweilzeit von 2½ Stunden kommt die Verseifungslösung in einen anderen Verseifungskessel, dessen Temperatur bei 100°C liegt. Nach einer mittleren Verweilzeit von 4 Stunden fliesst das Gemisch in einen Nachreaktor, wo es bei einer Verweilzeit von 3 Stunden bei 110°C ausreagiert. Bei gleichzeitigem Abdampfen von Ammoniak, Anilin und Wasser erhält man stündlich 695 Teile Natrium/Kaliumsalz des Phenylglycins vom Fp 140 bis 160°C (Zers.) und einer Reinheit von 90 Prozent (gemessen durch Hochdruck-Flüssigkeits-Chromatographie), entsprechend 625 Teile reinen Endstoff (86,4% der Theorie).

Beispiel 4 (Vergleich)

Umsetzung und Hydrolyse werden analog Beispiel 2 der DE-PS 1 543 342 durchgeführt. Man erhält stündlich 181 Teile Natrium/Kaliumsalz des Phenylglycins, die noch deutliche Verunreinigungen (Befund unter Verwendung der Hochdruck-Flüssigkeits-Chromatographie) aufweisen. Nach Befund unter Verwendung der Hochdruck-Flüssigkeits-Chromatographie liegt der Reingehalt bei 89 Prozent, entsprechend 161 Teilen reinen Endstoff (89% der Theorie).

**Patentanspruch**

Verfahren zur Herstellung von Alkalisalzen des Phenylglycins durch Umsetzung von Anilin mit Formaldehyd und Blausäure in Gegenwart von Wasser während 0,1 bis 4 Stunden bei einer Temperatur von 0 bis 80°C, wobei die Konzentration der Blausäure während der Reaktion nicht mehr als 0,9 Gewichtsprozent, bezogen auf das Reaktionsgemisch, beträgt und Hydrolyse des gebildeten Phenylglycinnitrils, dadurch gekennzeichnet, dass die Hydrolyse in Abwesenheit oder in Gegenwart von Anilin in einer Menge zwischen 0 und 0,2 Mol Anilin je Mol Phenylglycinnitril mit Alkalilaugen in einem Verhältnis von 1 bis 1,05 Mol Alkalihydroxid je Mol Phenylglycinnitril in Gegenwart von Alkoholen, aromatischen Kohlenwasserstoffen und/oder aliphatischen Kohlenwasserstoffen als organischem Lösungsmittel in einer Menge von 5 bis 200 Gewichtsprozent und von Wasser in einer Menge von 50 bis 500 Gewichtsprozent, bezogen auf Ausgangsanilin, durchgeführt wird.

**Claim**

A process for the preparation of alkali metal salts of phenylglycine by reacting aniline with formaldehyde and hydrogen cyanide in the presence of water for from 0.1 to 4 hours at from 0 to 80°C, the concentration of hydrogen cyanide in

the reaction mixture during the reaction being not more than 0.9 per cent by weight, and hydrolyzing the phenylglycinonitrile formed, wherein the hydrolysis is carried out in the absence of aniline or in the presence of from 0 to 0.2 mole of aniline per mole of phenylglycinonitrile, with an alkali metal hydroxide solution, using from 1 to 1.05 moles of alkali metal hydroxide per mole of phenylglycinonitrile, in the presence of from 5 to 200 per cent by weight, based on starting aniline, of an alcohol, aromatic hydrocarbon and/or aliphatic hydrocarbon as the organic solvent and in the presence of from 50 to 500 per cent by weight, based on starting aniline, of water.

**Revendication**

Procédé de préparation de sels de métaux alcalins du phényl-glycocolle par réaction de l'aniline pendant 0,1 à 4 heures à une température entre 0 et 80°C avec le formaldéhyde et l'acide cyanhydrique en présence d'eau, la concentration du mélange réactionnel en acide cyanhydrique ne dépassant pas 0,9% en poids durant la réaction, suivie de l'hydrolyse du nitrile du phényl-glycocolle formé, caractérisé en ce que l'hydrolyse est réalisée en présence de 5 à 200% en poids d'un solvant organique choisi parmi les alcools et les hydrocarbures aromatiques et(ou) aliphatiques et de 50 à 500% en poids d'eau par rapport à l'aniline de départ, à l'aide d'une lessive alcaline en une proportion de 1 à 1,05 mole d'hydroxyde de métal alcalin par mole de nitrile du phényl-glycocolle, éventuellement en présence d'aniline en une proportion pouvant aller jusqu'à 0,2 mole par mole de nitrile du phényl-glycocolle.